# EUROPEAN PATENT APPLICATION

(11) **EP 2 821 091 A1**
(43) Date of publication of application: **07.01.2015**
(21) Application number: 13755242.8
(22) Date of filing: 26.02.2013
(51) Int. Cl.: A61M 5/50, A61M 5/34, A61M 5/31

(54) **NEEDLE RETRACTABLE-TYPE SAFE SELF-DESTRUCTING SYRINGE**

(30) Priority: 29.02.2012 CN 201220071139 U
(71) Applicant: Wang, Zuyang, Shanghai 200336 (CN)
(72) Inventor: Wang, Zuyang, Shanghai 200336 (CN)
(74) Representative: Petraz, Gilberto Luigi
(86) International application number: PCT/CN2013/000189
(87) International publication number: WO 2013/127258

(57) **Abstract**

A needle retractable-type safe self-destructing syringe, which comprises a barrel body (1). A needle base body (3) is arranged within a barrel neck of an end part of the barrel body (1). The barrel neck (2) and the needle base body (3) form therebetween a flexible cushioning stopper mechanism (4). An asymmetric retracting-locking piece (10) having a flexibility feature is arranged at an end part of a syringe plunger (8). The flexibility feature implements engaging and locking with an outer sleeve-type retracting-locking component (5) at the lower part of the needle base body (3). In a stress environment of an asymmetric force, the needle base body (3) deflects to a side after being retracted into the barrel body (1), while an injection needle therefore swings in the direction of a nearly 45 degree angle to a side of the inner wall of the barrel body (1) and is locked by a neck part (2). This effectively prevents secondary injury caused by the needle being pushed out, and implements truly safe self-destruction of the syringe. The flexible asymmetric retracting-locking piece (10) is a plastic barb body; same is formed with the plunger (8) in a single mold-forming.

## Description

### BACKGROUND OF THE INVENTION

### FIELD OF THE INVENTION

The present invention relates to a technical field of medical devices, more particularly to a retractable disposable safety syringe with a pre-attached needle, and especially to the mechanical connection structure.

### DESCRPTION OF RELATED ART

Reference Document 1, Patent No.: ZL200710047077.4; Application Date: 2007.10.16; Issued No.: CN101411908; assignee: Shanghai Dingjiantang Biochemical Technology Co., Ltd. Reference Document 1 discloses "a retractable safety vaccine syringe". The retractable safety vaccine syringe comprises a barrel body in which a plunger is disposed, a rubber stopper disposed near an end of the plunger, a barrel neck of the barrel body through which a needle is disposed, and a needle tube disposed in the needle. It is characterized in that a side wall of a rod body of an end of the plunger is provided with a lateral opening. The lateral opening is connected with a vertical opening of the rod body of the end of the plunger. The lateral opening matches the vertical opening. An upper needle base is sleeved onto a lower needle base to form the needle. The lower needle base has a hollow cavity. The lower needle base is in a barrel shape. A lower needle base rib is disposed on an inner wall of an opening of an end of the lower needle base opposite the opening of the end of the lower needle base for disposing the upper needle base. An elastic member formed for connecting the lateral opening with the vertical opening is disposed on the end of the plunger. The plunder of the plunger has a specific elasticity. An upper part of the vertical opening is narrow, and a lower part thereof is wide. On the outside of the lateral opening, an outer wall of the rod body of an upper portion of the plunger protrudes beyond an outer wall of the rod body of a lower portion of the plunger. The out part of the lateral opening is higher, and the inner part thereof is low. The end of the plunger is curved. The upper needle base is in a barrel shape. A plurality of reinforcing ribs are disposed on an outer wall of an upper portion of the upper needle base. A flange ring of the upper needle base is disposed on an outer wall of the lower portion of the upper needle base. An outer diameter of the flange ring of the upper needle base is greater than the diameter (0.05mm-0.3mm) of the outer wall of the upper needle base. The lower needle base is in a barrel shape and has a hollow cavity. An annular locking recess of the lower needle base is disposed on the inner wall of the cavity of the lower needle base. The annular locking recess of the lower needle base matches the flange ring of the upper needle base. The inner diameter of an upper cavity of the annular locking recess of the lower needle base is smaller than the outer diameter (0.1mm-0.35mm) of the flange ring of the upper needle base. The inner diameter of the cavity of the lower part of the annular locking recess of the lower needle base is less than the outer diameter of the plunger of the lower part of the flange ring of the upper needle base. An annular flange rib of the lower needle base is disposed on the outer wall of the end opposite the end of the flange ring of the lower needle base. An upper stopping ring of the barrel neck and a lower stopping ring of the barrel neck are respectively disposed on the inner wall of the barrel neck of the barrel body. The distance between the upper stopping ring of the barrel neck and the lower stopping ring of the barrel neck is 0.3mm-1.5mm. The upper spacing ring of the barrel neck protrudes beyond an inner wall between the upper stopping ring of the barrel neck and the lower stopping ring of the barrel neck by 0.1mm-0.6mm. The lower spacing ring of the barrel neck protrudes beyond the inner wall between the upper stopping ring of the barrel neck and the lower stopping ring of the barrel neck by 0.02mm-0.25mm. The inner diameter of the cavity of the upper part of the barrel body at the upper stopping ring of the barrel neck is greater than an inner diameter of the cavity of the barrel body between the upper stopping ring of the barrel neck and the lower stopping ring of the barrel neck. The inner diameter of the cavity of the barrel body between the upper stopping ring of the barrel neck and the lower stopping ring of the barrel neck is less than the inner diameter of the cavity of the barrel body below the lower spacing ring of the barrel neck. The inner diameter of the cavity of the barrel body between the upper stopping ring of the barrel neck and the lower stopping ring of the barrel neck is smaller than the outer diameter of the annular flange rib of the lower needle base by 0.1mm-0.5mm. A stopping rib of the barrel body is disposed on the inner wall of the cavity of the barrel body opposite the end of the barrel neck. The stopping rib can a linear annular rib, a vertical rib, an annular rib formed from a plurality of vertical ribs, or an annular rib formed from multiple protrusions. An inner flange rib of the barrel body is disposed on the inner wall of the barrel body below the stopping rib of the barrel body. A distance between the stopping rib of the barrel body and the inner flange rib of the barrel body is greater than the thickness of the annular stopping piece. The inner diameter of the stopping rib of the barrel body is smaller than the diameter of the annular stopping piece.

Reference Document 2, Patent No.: ZL200720067715.4; Application Date: 2007.03.08; Issued No.: CN201076648; Assignee: Shanghai Dingjiantang Biochemical Technology Co., Ltd. Reference Document 2 discloses "a plunger with a metal hook". The plunger with a metal hook comprises a plunger body of which a columnar locking member is disposed on an upper end, and the columnar locking member sleeved in a rubber piston. It is characterized in that a mounting hole is disposed outside the rubber piston, and a hook is disposed in the mounting hole.

In the current safety syringes, a function for retracting a needle is carried out via the hook so as to perform injections safely. The hook is generally made from a metal piece or a plastic piece. The metal piece is required to be manufactured so as to match or be assembled on the plunger. The manufacturing process is complicated and difficult, and lacks stability. Not only is the manufacturing process cost increased, but the metal piece and the assembly process thereof increase the uncertainty of product safety and product quality. The current plastic hook still has structural defects or deficiencies, thereby leading to safety risks during use. Though the current plastic locking design can fulfill the needle-retracting function, after the needle is retracted, the needle still remains on the regular track during use. There is a risk for the needle to be pushed out again, resulting in the second injury to the user.

In addition, the current retractable disposable safety syringe with a single needle base and the safety syringe both have a common problem: how to achieve locking stability between an inner needle base and a barrel body, so that the strength meets stability requirements. Furthermore, the pulling force for retracting the needle base cannot be too great in order to facilitate use. The rigid locking structure can achieve a stable locking effect between the inner needle base and the inner wall of the barrel body, but it is difficult or even impossible to meet the requirement for convenient use at the same time. The instability of plastic materials and their propensity for being easily affected by environmental temperature changes lead to poor stability of the products with rigid locking structures. It is difficult to control the quality and precision during the production process, and is hard to mass produce.

As mentioned above, it is still necessary to further improve the locking structure between the plunger, the inner needle base, and the barrel body of the current retractable syringe, thereby further improving safety and stability during use. The manufacturing process can be simplified, and the manufacturing cost can be reduced.

### BRIEF SUMMARY OF THE INVENTION

The object of the present invention is to provide a retractable disposable safety syringe with a pre-attached needle. An inner needle base of the syringe interlocks a barrel body of the syringe via an elastic dampening mechanism. The locking and releasing effects therebetween are achieved by utilizing the expansion force generated by the deformation of the dampening gap. The elastic dampening mechanism can be formed at the inner needle base or on an inner wall of the barrel body. Meanwhile, an elastic dampening locking mechanism is formed at the inner needle base and in the barrel body. An end part of the plunger is provided with an asymmetrical elastic retractable locking member. The asymmetrical elastic retractable locking member can be locked within the cavity of the needle base of the syringe through interlocking. After the needle base is retracted into the barrel body of the syringe, the needle base can deflect toward one side under a force from an asymmetrical elastic structure. The needle therefore inclines in the direction of a nearly 45 degree angle to a side of the inner wall of the barrel body, and is locked by the barrel neck of the barrel body, so that the needle and the needle base cannot be pushed out of the barrel neck of the barrel body again. The needle is prevented from being pushed out again, causing the second injury. Safe operation and self-destruction of the syringe are realized, thereby overcoming defects and deficiencies in the prior art.

In order to achieve above object, the technical solution of the present invention is to provide a retractable disposable safety syringe with a pre-attached needle. The retractable disposable safety syringe with the pre-attached needle comprises a barrel body, a needle base body disposed in a barrel neck of an end of the barrel body, a plunger disposed in the barrel body, and a rubber piston disposed near an end of the plunger. It is characterized in that an upper part of the needle base body is located in the barrel neck. A middle part of the needle base body forms an elastic sealing stopper. A lower part of the needle base body is connected with a sleeve-type retractable locking member. A surface of the elastic sealing stopper is provided with an annular dampening gap. An annular sealing-locking part is formed on an outer wall of the elastic sealing stopper outside the annular dampening gap. An asymmetrical elastic retractable locking member is disposed on an end of the plunger.

The present invention discloses a retractable disposable safety syringe with a pre-attached needle. The asymmetrical elastic retractable locking member is disposed at the end of the plunger of the retractable disposable safety syringe with the pre-attached needle. The combination of an asymmetrical structure and an elastic mechanism can ensure that the asymmetrical elastic retractable locking member can be smoothly inserted into the cavity of the needle base. The interlocking between both sides of the asymmetrical locking member and a locking part of the sleeve-type retractable locking member is realized by the elastic expansion mechanism. Since an annular recess in the cavity of the sleeve-type retractable locking member also has the elastic and dampening characteristic, when the sleeve-type retractable locking member is being pushed to contact the asymmetrical elastic retractable locking member, both have dampening deformation correspondingly, so that the asymmetrical elastic retractable locking member gets stuck in the cavity of the sleeve-type retractable locking member, and locked with the sleeve-type retractable locking member when the asymmetrical elastic retractable locking member is retracted. After the needle base is retracted into the barrel body of the syringe, the needle is deflected to a side under the asymmetrical force. The needle inclines in the direction of a nearly 45 degree angle to a side of the inner wall of the barrel body, and is locked by the barrel neck of the barrel body, so that the needle and the needle base cannot be pushed out of the barrel neck of the barrel body again. The needle is prevented from being pushed out again, causing the second injury. Safe operation and self-destruction of the syringe are realized. In the embodiment of the present invention, the asymmetrical elastic retractable locking member is a plastic hook. The design principle of a subtle combination between an asymmetrical structure is used and an elastic structure in the plastic hook, and the specific angles and sizes are utilized and designed, so that the plastic hook has the elastic dampening and locking functions. The above mentioned asymmetrical elastic retractable locking member and the plunger are formed integrally into one piece. The whole elements are molded integrally. Independent manufacture or follow-up assembly is not required. The manufacturing cost is efficiently reduced. The assembly process and procedure are simplified and reduced. It is simple to use and operate the product. The operating state is steady. The interlocking and locking effect between the asymmetrical elastic retractable locking member on the end of the plunger and the cavity of the needle base is excellent. The integral structure of the product is reasonable. The idea is ingenious. The product can be used, applied and promoted easily. In comparison with the design and the technique of the current product, the present invention has significant technical breakthroughs, and is safe and practicable.

### BRIEF DESCRIPTION OF THE DRAWINGS

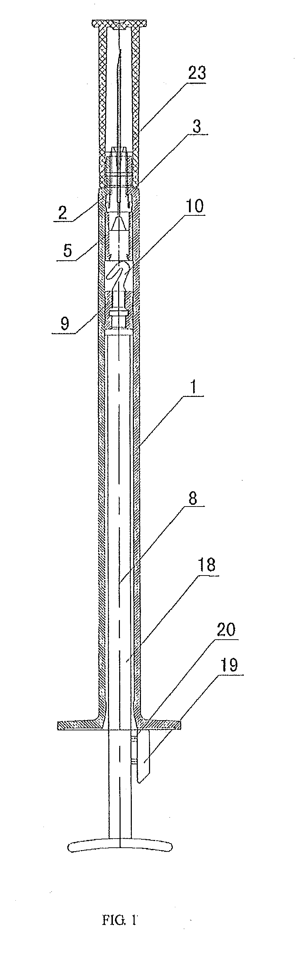

FIG. 1 is a schematic diagram of the present invention.

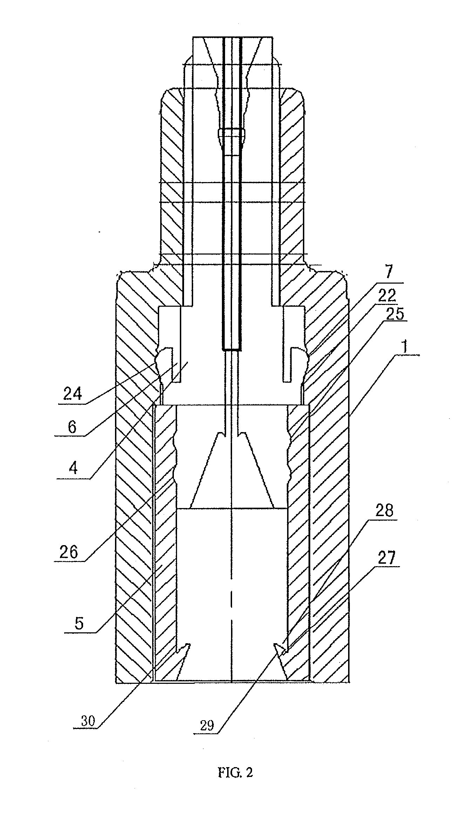

FIG. 2 is an assembly diagram of a needle base body and a sleeve-type retractable locking member of the present invention.
FIG. 3 is a schematic diagram of the hook of the present invention.
FIG. 4 is a diagram of a first use state of the present invention.

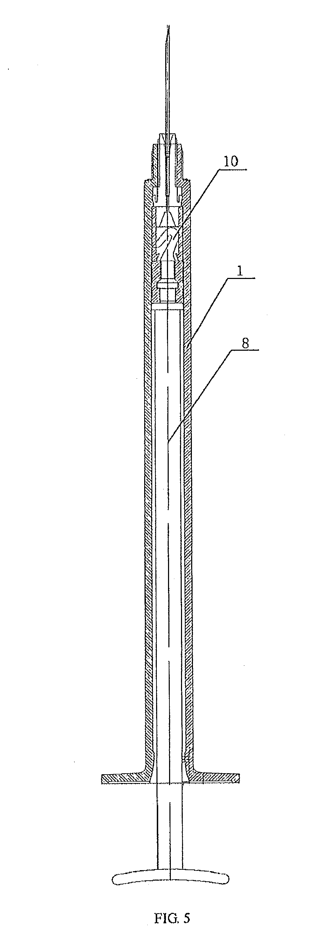

FIG. 5 is a diagram of a second use state of the present invention.

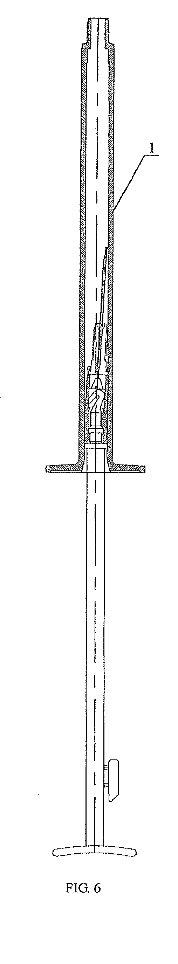

FIG. 6 is a diagram of a third use state of the present invention.

### DETAILED DESCRIPTION OF THE PREFERRED EMBODIMENT

Hereinafter, preferred embodiments of the present disclosure will be described in detail with reference to the accompanying drawings.

The present invention is a retractable disposable safety syringe with a pre-attached needle, which comprises a barrel body 1, a needle base body 3 disposed in a barrel neck 2 of an end of the barrel body 1, a plunger 8 disposed in the barrel body 1, and a rubber piston 9 disposed near an end of plunger 8. It is characterized in that an upper part of the needle base body 3 is located in the barrel neck 2. A middle part of the needle base body 3 forms an elastic sealing stopper 4. A lower part of the needle base body 3 is connected with a sleeve-type retractable locking member 5. A surface of the elastic sealing stopper 4 is provided with an annular dampening gap 6. An annular sealing-locking part 7 is formed on an outer wall of the elastic sealing stopper 4 outside the annular dampening gap 6. An asymmetrical elastic retractable locking member 10 is disposed with on an end of the plunger 8. The needle base body 3 and the barrel body 1 can be interlocked stably and released easily due to the elastic sealing stopper 4.

An elastic mechanism is formed on one side or both sides of the asymmetrical elastic retractable locking member. In addition, an outer side part of the elastic mechanism on one side of the asymmetrical elastic retractable locking member protrudes beyond a side wall of a junction of the asymmetrical elastic retractable locking member and the plunger 8, thereby ensuring the locking effect and the deflection function between the asymmetrical elastic retractable locking member and a needle.

In the detailed embodiment, the asymmetrical elastic retractable locking member 10 is a hook. The hook is made from a plastic material. The hook and the plunger 8 are formed integrally into one piece, are molded integrally, or are separately form followed by being assembled together.

In the detailed embodiment, the hook can also be assembled at the end of the plunger via other assembly structures.

In the detailed embodiment, a base portion 11 is formed on a lower end of the hook, a first arm 12 deflected to one side is formed on an upper part of the base portion 11. A concave curved angle 13 or corner is defined by an outer sidewall of a junction between the first arm 12 and the base portion 11. The concave curved angle 13 is ranged from 45 to 179 degrees. A lower end of the first arm 12 is connected with an upper end of the base portion 11. An upper end of the first arm 12 is connected with an end of a second arm 14. Another end of the second arm 14 is suspended. A curved surface 15 is formed on an outer side of a junction between the first arm 12 and the arm 14. An inner angle 17 of 2-90 degrees is defined by an inner side of the junction between the first arm 12 and the second arm 14. A width of an opening between the suspended end of the second arm 14 and the first arm 12 is 0.5mm-40mm. The length of the first arm 12 is 0.5mm-50mm, and the length of the second arm 14 is 0.5mm-60mm. A structure designed in such a way can ensure the locking effect and the deflection function between the hook and the needle.

In the detailed embodiment, four reinforcing ribs 18 arranged in a cross shape and at equal angles are formed on the plunger 8. A stopping piece 19 is disposed on an outer edge of the reinforcing ribs 18. The stopping piece 19 is connected with the reinforcing ribs 18 via at least two contact points 20. After the syringe is assembled, a top end of the stopping piece is just against on the lower end of the barrel body thereby preventing the plunger from moving forwardly and the syringe from being wasted when the syringe is under an external force before use. In use, the stopping piece only needs to be bent the stopping piece along the contact points, so as to remove the stopping piece from the reinforcing ribs.

In the detailed embodiment, the annular dampening gap 6 is an annular groove. A height from an opening of the groove to a bottom of the groove is 0.05mm-4mm. A width of the annular dampening gap 6 is 0.05mm-4mm. The distance from an outer groove wall of the annular dampening gap 6 to a top of a curved surface of the annular sealing-locking part 7 is 0.1mm-6mm. The distance from the opening of the outer groove wall of the annular dampening gap 6 to the outer wall of the annular sealing-locking part 7 is 0.1mm-6mm. The distance from the bottom of the outer groove wall of the annular dampening gap 6 to the outer wall of the annular sealing-locking part 7 is 0.1mm-6mm. A surface of the annular sealing-locking part 7 is a curved surface. The distance from the top of the curved surface of the annular sealing-locking part 7 to an upper edge of the curved surface is less than the distance from the top of the curved surface of the annular sealing-locking part 7 to a lower edge of the curved surface. An annular recess 22 is defined by the outer wall of elastic sealing stopper 4 of a lower part of the annular sealing-locking part 7.

In the detailed embodiment, the annular recess 22 is in the same horizontal plane as a groove bottom of the annular dampening gap 6, or higher than a groove bottom of the annular dampening gap 6.

In the detailed embodiment, the exterior of the barrel neck 2 of the barrel body 1 is covered by a barrel body cap 23.

In the detailed embodiment, an annular sealing recess 24 matching the annular sealing-locking part 7 is disposed on an inner wall of the barrel neck 2 of the barrel body 1. The depth of the annular sealing recess 24 is 0.01mm-4mm. The height of the annular sealing recess 24 is 0.01mm-6mm, and a recess bottom of the annular sealing recess 24 is a curved surface.

In the detailed embodiment, at least a flange ring 25 is formed on an outer wall of the needle base body 3, and an annular stopping groove 26 matching the flange ring 25 is disposed on an inner wall of the sleeve-type retractable locking member 5.

In the detailed embodiment, the sleeve-type retractable locking member 5 has a hollow inner cavity. An annular locking anchor 27 is formed on the inner wall of the cavity near an opening of a lower end of the sleeve-type retractable locking member 5.

In the detailed embodiment, an annular recess 28 is formed on an upper part of the annular locking anchor 27. A thickness of an inner sidewall 29 of the annular recess 28 is 0.01mm-3mm. An angle of 2-90 degrees is formed between the inner side wall of the annular recess 28 and an outer sidewall of the annular recess 28. An inner diameter of an upper end of the inner side wall of the annular recess 28 is 1mm-80mm. The width of an opening of the annular recess 28 is greater than the width of a bottom of the annular recess.

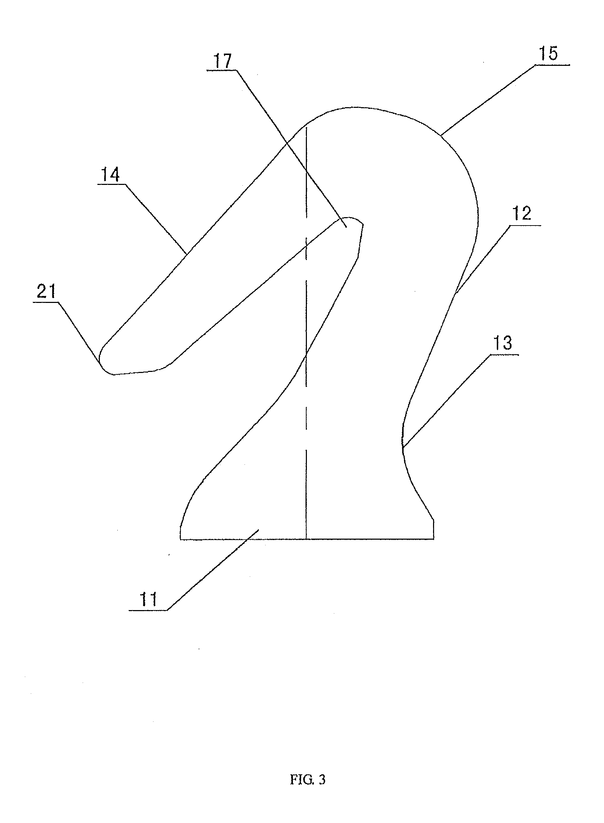

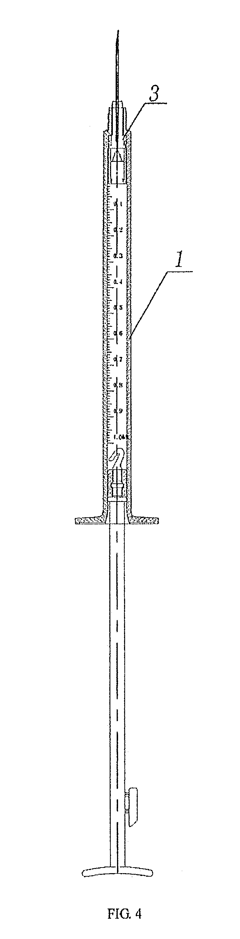

In practical operation, as shown in FIG. 3 to FIG. 4, after the liquid is drawn, the injection begins, and the plunger is slowly pushed. The total width of the asymmetrical elastic retractable locking member is greater than the inner diameter of the annular recess of the sleeve-type retractable locking member. One side of the asymmetrical elastic retractable locking member is deformed due to the contact between the asymmetrical elastic retractable locking member and the sleeve-type retractable locking member, so that the width is narrowed, and then the asymmetrical elastic retractable locking member enters the cavity of the sleeve-type retractable locking member. After the asymmetrical elastic retractable locking member enters the cavity, and the force is removed, the asymmetrical elastic retractable locking member restores to its original state. Meanwhile, the injunction is finished, and the plunger is pulled backwards so that the side of the asymmetrical elastic retractable locking member (the suspended end of the second arm of the hook) is just stuck in the annular recess of the sleeve-type retractable locking member. Under the pulling force of the plunger, the pulling force is transferred to the barrel body through the sleeve-type retractable locking member. The annular dampening gap of the elastic sealing stopper is deformed by the pulling force. The annular sealing-locking part slides out from the annular sealing recess of the barrel body while the annular dampening gap is being narrowed down. Finally, the whole needle is pulled into the cavity of the barrel body. The needle is deflected to one side under the action of the asymmetrical elastic retractable locking member, and deviated from the regular displacement track, so that the needle cannot be re-assembled into the barrel neck, thereby realizing safe operation and self-destruction of the syringe.

The above content describes the present invention in combination with the detailed preferred embodiment method. However, it should not be considered that the detailed embodiment of the present invention is only limited to the above description. A person of ordinary skill in the art, without departing from the concept of the present invention, may make many simple derivations or alternations, which should be deemed within the scope of the present invention.

## Claims

1. A retractable disposable safety syringe with a pre-attached needle, comprising a barrel body (1), a needle base body (3) disposed in a barrel neck (2) of an end of the barrel body (1), a plunger (8) disposed in the barrel body (1), and a rubber piston (9) disposed near an end of plunger (8), **characterized in that**: an upper part of the needle base body (3) is located in the barrel neck (2), a middle part of the needle base body (3) forms an elastic sealing stopper (4), a lower part of the needle base body (3) is connected with an sleeve-type retractable locking member (5), a surface of the elastic sealing stopper (4) is provided with an annular dampening gap (6), an annular sealing-locking part (7) is formed on an outer wall of the elastic sealing stopper (4) outside the annular dampening gap (6), and an asymmetrical elastic retractable locking member (10) is disposed on an end of the plunger (8).

2. The retractable disposable safety syringe with the pre-attached needle as claimed in claim 1, **characterized in that**: the asymmetrical elastic retractable locking member (10) is a hook, the hook is made from a plastic material, and the hook and the plunger (8) are formed integrally into one-piece, molded integrally, or separately formed followed by being assembled together.

3. The retractable disposable safety syringe with the pre-attached needle as claimed in claim 2, **characterized in that**: a base portion (11) is formed at a lower end of the hook, a first arm (12) deflected to one side is formed on an upper part of the base portion (11); a concave curved angle (13) or corner is defined by an outer sidewall of a junction between the first arm (12) and the base portion (11), the concave curved angle (13) is ranged from 45 to 179 degrees, a lower end of the first arm (12) is connected with an upper end of the base portion (11), an upper end of the first arm (12) is connected with an end of a second arm (14), another end of the second arm (14) is suspended, a curved surface (15) is formed on an outer side of a junction between the first arm (12) and the arm (14), an inner angle (17) of 2-90 degrees is defined by an inner side of the junction between the first arm (12) and the second arm (14), a width of an opening between the suspended end of the second arm (14) and the first arm (12) is 0.5mm-40mm, the length of the first arm (12) is 0.5mm-50mm, the length of the second arm (14) is 0.5mm-60mm; a tip (21) is formed on the suspended end of the second arm (14), and the suspended end of the second arm (14) protrudes beyond an outer edge of the base portion (11).

4. The retractable disposable safety syringe with the pre-attached needle as claimed in claim 1, **characterized in that**: four reinforcing ribs (18) arranged in a cross shape and in equal angles are formed on the plunger (8), a stopping piece (19) is disposed on an outer edge of the reinforcing ribs (18), and the stopping piece (19) is connected with the reinforcing ribs (18) via at least two contact points (20).

5. The retractable disposable safety syringe with the pre-attached needle as claimed in claim 1, **characterized in that**: the annular dampening gap (6) is an annular groove, a height from an opening of the groove to a bottom of the groove is 0.05mm-4mm, a width of the annular dampening gap (6) is 0.05mm-4mm, the distance from an outer groove wall of the annular dampening gap (6) to a top of a curved surface of the annular sealing-locking part (7) is 0.1mm-6mm, the distance from the opening of the outer groove wall of the annular dampening gap (6) to the outer wall of the annular sealing-locking part (7) is 0.1mm-6mm, the distance from the bottom of the outer groove wall of the annular dampening gap (6) to the outer wall of the annular sealing-locking part (7) is 0.1mm-6mm, a surface of the annular sealing-locking part (7) is a curved surface, the distance from the top of the curved surface of the annular sealing-locking part (7) to an upper edge of the curved surface is less than the distance from the top of the curved surface of the annular sealing-locking part (7) to a lower edge of the curved surface, and an annular recess (22) is defined by the outer wall of elastic sealing stopper (4) of a lower part of the annular sealing-locking part (7).

6. The retractable disposable safety syringe with the pre-attached needle as claimed in claim 1, **characterized in that**: the annular recess (22) is in the same horizontal plane as a groove bottom of the annular dampening gap (6), or higher than a groove bottom of the annular dampening gap (6).

7. The retractable disposable safety syringe with the pre-attached needle as claimed in claim 1, **characterized in that**: the exterior of the barrel neck (2) of the barrel body (1) is covered by a barrel body cap (23), an annular sealing recess (24) matching the annular sealing-locking part (7) is disposed on an inner wall of the barrel neck (2) of the barrel body (1), the depth of the annular sealing recess (24) is 0.01mm-4mm, the height of the annular sealing recess (24) is 0.01mm-6mm, and a recess bottom of the annular sealing recess (24) is a curved surface.

8. The retractable disposable safety syringe with the pre-attached needle as claimed in claim 1, **characterized in that**: at least a flange ring (25) is formed on an outer wall of the needle base body (3), and an annular stopping groove (26) matching the flange ring (25) is disposed on an inner wall of the sleeve-type retractable locking member (5).

9. The retractable disposable safety syringe with the pre-attached needle as claimed in claim 1, **characterized in that**: the sleeve-type retractable locking member (5) has a hollow inner cavity, and an annular locking anchor (27) is formed on the inner wall of the cavity near an opening of a lower end of the sleeve-type retractable locking member (5).

10. The retractable disposable safety syringe with the pre-attached needle as claimed in claim 1, **characterized in that**: an annular recess (28) is formed on an upper part of the annular locking anchor (27), a thickness of an inner sidewall (29) of the annular recess (28) is 0.01mm-3mm, an angle of 2-90 degrees is formed between the inner side wall of the annular recess (28) and an outer sidewall of the annular recess (28), and an inner diameter of an upper end of the inner side wall of the annular recess (28) is 1mm-80mm.

11. The retractable disposable safety syringe with the pre-attached needle as claimed in claim 1, **characterized in that**: the width of an opening of the annular recess (28) is greater than the width of a bottom of the annular recess (28).
